Europäisches Patentamt

**(19)** European Patent Office

Office européen des brevets

**(11)** **EP 0 428 793 B1**

**(12)** EUROPEAN PATENT SPECIFICATION

**(45)** Date of publication and mention
of the grant of the patent:
**31.01.1996 Bulletin 1996/05**

**(51)** Int. Cl.$^6$: **A61B 5/107**, A61B 5/11

**(21)** Application number: **89311854.7**

**(22)** Date of filing: **16.11.1989**

**(54)** **Device for measuring the drop of the uterus**

Vorrichtung zur Messung der Gebärmuttersenkung

Instrument pour mesurer la descente de l'uterus

**(84)** Designated Contracting States:
**AT BE CH DE ES FR GB GR IT LI NL SE**

**(43)** Date of publication of application:
**29.05.1991 Bulletin 1991/22**

**(73)** Proprietor: **Bellas, Gabriel Antonio Santos**
**El Marques Caracas Estado Miranda (VE)**

**(72)** Inventor: **Bellas, Gabriel Antonio Santos**
**El Marques Caracas Estado Miranda (VE)**

**(74)** Representative: **Pacitti, Pierpaolo A.M.E. et al**
**Glasgow G5 8QA (GB)**

**(56)** References cited:
**DE-B- 2 650 252**       **DE-C- 3 330 921**
**FR-A- 2 622 428**       **GB-A- 2 035 097**
**US-A- 4 489 732**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

### FIELD OF THE INVENTION

This invention is related to the field of medicine and refers to a method for measuring the drop of the uterus and/or vaginal vault in normal patients or with uterine prolapse. It is especially related to an instrument that basically involves a cylindrical plunger mesured in centimeters with a sliding internal end within a concentric cover with top at the level of the external genitals and which has an adjustable scale to measure drop in centimeters, which is introduced into the patient end to end with her uterine cervix, and permits measuring the vaginal depth and when the patient pushes, displaces the plunger, the displacement can be measured on a scale previously adjusted to zero; and the results of which permit determining the level of uterine prolapse and diagnosing the normal or pathological condition of a patient.

### SUMMARY OF THE INVENTION

The objective of this invention is to provide an instrument for ambulatory use for easy and rapid diagnosis. Until the present time, diagnoses to define the state of uterine prolapse require time and are expensive, necessitate applications of X-rays, tracing plates, and contrasts applied by enemas and/or probes. The principal objective is to be able to detect prolapses in their primary phase (type I), which generally are not detected with current known techniques, especially the physical examination. Another objective is to minimize the possible surgical interventions that in a very high proportion are performed in patients operated for Burch or Marshall-Marchette-Krantz type urinary incontinence of strength which alter the normal vaginal axis.

The use of this instrument for diagnosis: is of special utility in cases which increase the intra-abdominal pressure or the uterine position and their ligaments are altered in patients such as: workers who stand, obese patients, patients with chronic pulmonary diseases, menopausal patients, patients with uteruses in retrodeviation, endometriosis, inflammatory pelvic diseases, multiparas, vaginal prolapse and urinary incontinence of strength or background of application of forceps, therefore it permits the EARLY DIAGNOSIS of histerocele or type I uterine prolapse, when it can easily pass a clinical examination without being detected. Symmonds et. al. (1 to 14) of the Mayo Clinic has studied the period of operation and prolapse of the vaginal vault, finding a 63% error rate in diagnosis or surgery between 0 and 10 years from hysterectomy.

For Rust (12), Yates (13), Randall and Nicholls (5) and others: the high incidence of urinary incontinence and vaginal prolapse subsequent to Hysterectomies are an indication of the diagnostic failure of uterine prolapse prior to this intervention.

The instrument also serves for Post-Operatory controls of the surgical techniques that are utilized.

1. It permits measuring the vaginal depth (coital function).
2. It evaluates over time, by successive and periodic controls the effectivity of the technique used, according to the level of prolapse of the patient.

In these surgical series, a particular and original technique is used in which excellent results of the same quantified by this methbd can be appreciated.

### PRIOR ART

This invention is related to a methbd for measuring the decline of the uterus and/or vaginal vault in women with uterine prolapse, through a measuring instrument. In the medical literature no method exists which permits the measurement of the ligaments supporting the uterus or vaginal vault in normal or pathological (prolapses) cases.

There are functional anatomical clinical studies realized by MIOGRAPHY realized by Berglass and Rubin (ref. 01) in 1953, who studied the pelvic muscular supporting floor, especially the anus elevator through which the urethra, vagina and rectum cross, describing the tonicity of this muscle as a primordial supporting factor. RADIOLOGICAL studies of several authors, Richter K. (ref. 02, 03) in 1966 and 1967; Nichols D. Milley and Randall (ref. 04, 05) in 1970 and 1971 emphasize the importance of the anus elevator and its tonicity in the support of the uterus in the face of effort, acting as a top of the latter. Bethoux A. (ref. 06) obtains similar results but emphasizes the role of the supporting ligaments. All these authors emphasize the VAGINAL AXIS directed toward the 3rd or 4th vertebra of the sacrum changing the classical anatomical conception. All these studies of Functional Radiological anatomy offered a new concept on the physiopathology of the supporting mechanisms of the uterus. These investigations to detect uterine prolapses imply the introduction of means of contrast by iodized or barytic X rays in the bladder, probe and enema in the rectum and in the vagina in the form of creams, some with introduction of radio-opaque probes within the uterine vault, and other with plaster vaginal casts.

Other studies such as COLPOGRAPHY, which is an isolated visualization of the vagina have been performed for the control of the cases operated for prolapse of the vaginal vault.

Ridley Y. (ref. 7), Richter K. (ref. 03) and Nagata I. Kato K. (ref. 08) in 1976, 1981 and 1985.

COLPOTONOMETRY has been used by Semm and Penning cited by Pschyrembel (ref. 09) in 1973 and permits the registry of the volume of the vagina and the muscular forces of the pelvic floor, defining a gymnastic or surgical treatment for the vaginal volume and the muscular tone of its walls.

This invention is inspired in the pathological part: in the anatomical experiment performed in 1935 by William Mengert, who realized in different cadavers of women without prolapse cuts or cross-sections of different ligamental pairs and position of the uterus and observed, with measurements in centimeters with a clamp adapted to the cervix of the uterus, a pulley, and a ruler, the displacements that occur when each supporting ligament is cut. He points out that the muscular pelvic floor was never cut and in spite of it being intact, it did not avoid the experimental prolapse of the uterus (ref. 10). This antecedent lays the anatomical bases, measuring or quantifying in centimeters the importance of each supporting ligament of the uterus and vagina.

## STATEMENT OF THE INVENTION

According to the present invention there is provided an intra-vaginal diagnostic instrument comprising a plunger for insertion into the vagina of a patient, a sleeve within which the plunger is slidable and a scale which co-operates with an indicating means indicating the movement of the plunger in relation to the sleeve characterised in that the plunger has a first end which is placeable against the cervix of the uterus or the vaginal vault of the patent in which position the indicating means is adjustable to indicate an initial reading on the scale wherein the plunger is moveable by a force exerted by the patient on the first end of the plunger providing a subsequent reading of the indication means on the scale.

Preferably, the sleeve has a projection for location against the external entrance to the vagina.

The indicating means may be in the form of a notch provided on the plunger.

Alternatively, the indicating means may be in the form of an end of the sleeve distal from the entrance to the vagina.

As a further alternative, the indicating means may have a shaft having a dentated part which when moved axially due to movement the plunger, moves a needle in a radial direction on a scale, wherein the scale and the needle are attached to the sleeve. The plunger may be disposable and placeable end to end with the shaft.

Preferably, the scale indicates a movement of the plunger of at least 8 cm.

## BIBLIOGRAPHIC REFERENCES

01. Berglas, G.. & Rubin, I.C. (1953) "Study of the Supportive structures of the uterus by levator miography" Surg. Gynecol. Obstet., 97:677.

02. Richter, K. (1966) "Lebendige Anatomia der vagina. Geburts u Frauenh 26:1213.

03. Richter, K.L. & Albrich, W. (1981) Resultados a largo plazo siguientes a la fijación de la vagina en los ligamentos sacroespinales por Vía Vaginal. Am. J. Obset. Ginecol., 141:511-816.

04. Nichols, O.H.: Milley, P.S. & Randall, C.L. (1970) "Significance of restauration of normal vagine depth and axis". Obstet, Ginecology, 36:251-256.

05. Randall, C.L.; Nichols, F. & Nichols, O. (1971): Tratamiento Quirúrgico de la Inversión Vaginal. Obset. Ginecol., 38(3):327-332.

06. Betohoux, A., 1972 "Anatomía Funcional" cap. XXXI, in: Robert, H.G. Tratado de Técnica Quirúrgica Volume XIV. 1st ed. Barcelona, Toay, Masson. S.A. 1972.

07. Ridley, J.H. (1976): Una suspensión compuesta de la cúpula vaginal usando vendas Am. J. Obtet. Ginecol. 126:590-596.

08. Nagata, I.; Kato, K.; Faruya, K. & Kuki E. (1985): Reparación abdominal del prolapso vaginal y el resultado pos-operatorio para juzgar por un sistema de rayos y colpografía por rayos X ARCIA Ginecólogo Alemania, 237(1):11-7.

09. Psychyrembel W. "Ginecología Práctica." Ed. Alhambra S.A. 1973:377.

10. Mengert, W.F. (1935): Mechanics of Uterine Support and Position. Am. J. Obset. Ginecol. 775-782.

11. Symmonds, R.E. & Pratt, J.H. (1960): Prolapso Vaginal después de la histerectomía Am. J. Obstet. Ginecol., 61:248-252.

12. Rust, J.A., Botte, J.M. and Howlett, R.J., 1976. Prolapse of the vagina vault. Am. J. Obstet. Gynecol 125(6):768-776.

13. Yates, M.J. (1975) An Abdominal Approach to the repair of post-histerectomy vaginal inversion B.r. J. Obstet gynecol 82:817-819.

14. Symmonds, R.E., Williams, T.J., Lee, R.A.E., Webb, M.J. (1981) Post Histerotomy Enterocele anal vaginal Vault prolapse. Am.J. Obset Gynecol., 140:852-859.

## BRIEF DESCRIPTION OF THE DRAWINGS

As a way to illustrate the invention and with the purpose of clarifying this report, figures are attached which illustrate in a descriptive but not an all-encompassing way the scope of the ideas presented here; in respect to the instrument and method.

**FIGURE 1**.- Lateral and front views of the instrument of the invention with sliding measuring ring.
**FIGURE 2**.- Lateral and front views of the principal plunger.
**FIGURE 3**.- Lateral and front views of the cover of the plunger with its vulva end.
**FIGURE 4**.- Lateral and front views of the graduated measuring ring.

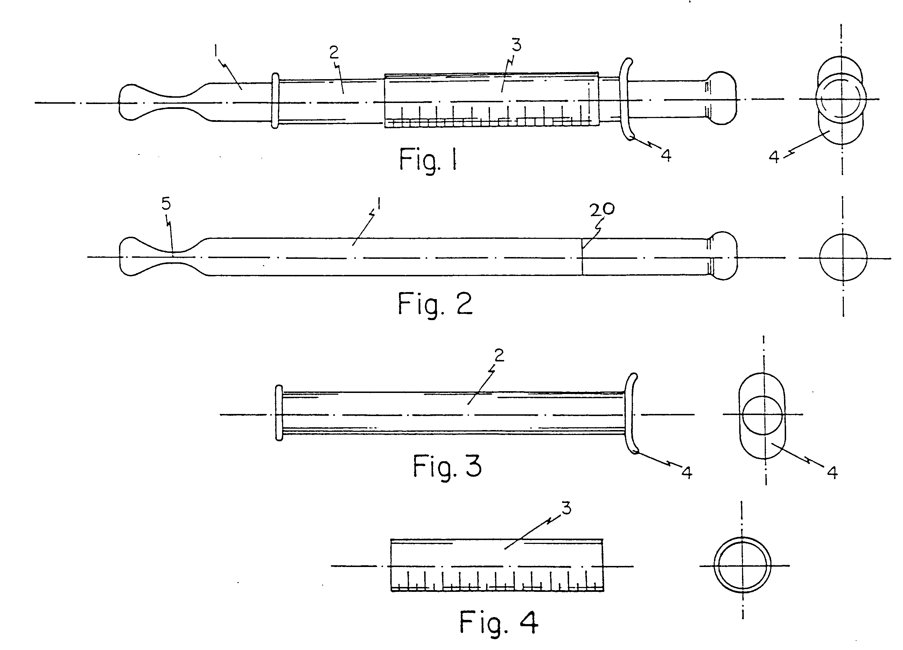

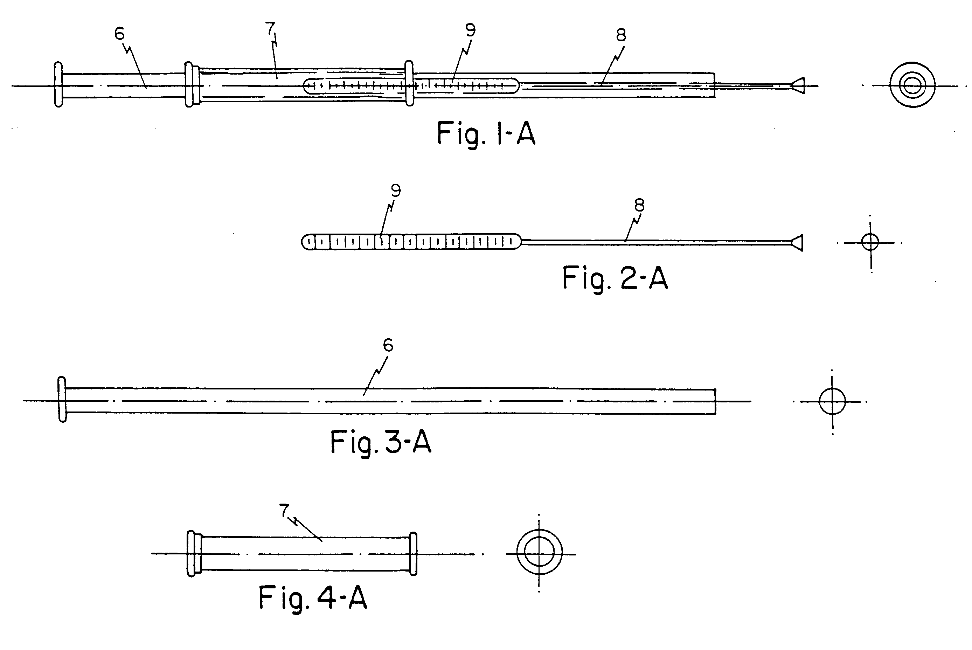

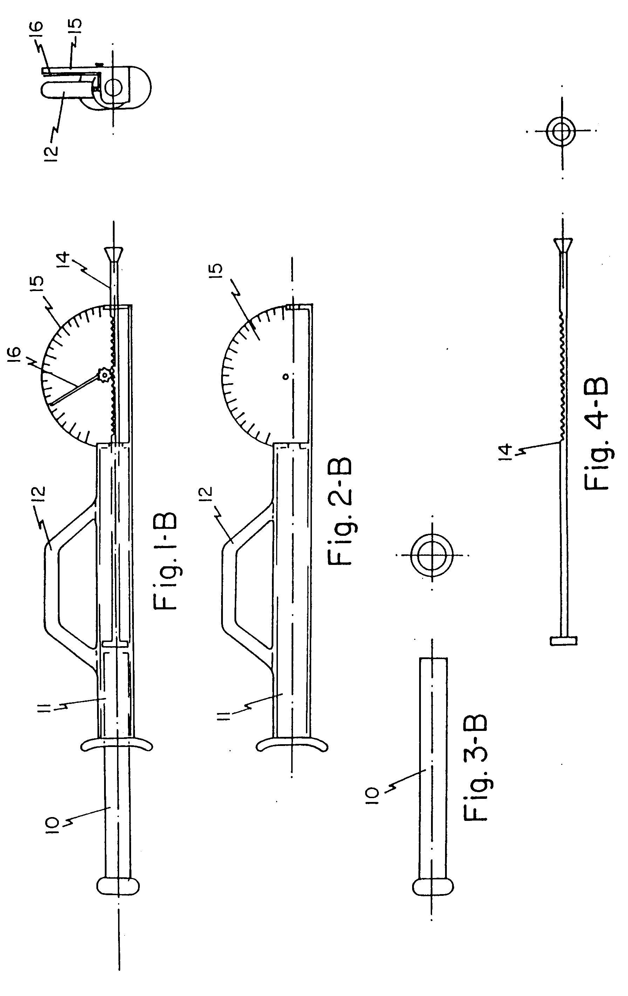

**FIGURE 1A**.- Lateral and front views of a variation of the instrument of the invention with a movable graduated measuring blade in the inside of the principal plunger.
**FIGURE 2A**.- Lateral and front views of the graduated measuring blade.
**FIGURE 3A**.- Lateral and front views of the principal plunger.
**FIGURE 4A**.- Lateral and front views of the concentric cylindrical cover of the plunger.
**FIGURE 1B**.- Lateral and front views of another variation of the instrument of the invention which includes a cover with an integral handle for easy handling and a graduated semicircular dial with a measuring needle.
**FIGURE 2B**.- Lateral view of the cover.
**FIGURE 3B**.- Lateral and front views of the section of the plunger for intravaginal insertion.
**FIGURE 4B**.- Lateral and front views of the plunger with its meshed portion for connection to the measuring needle in the semicircular dial.
**FIGURE 5**.- Sectional drawing which shows the utilization of the instrument within the anatomical region of a patient, with adjustment to zero of the instrument.
**FIGURE 6**.- Sectional drawing, showing a particular measurement with the instrument in a patient.

According to the mentioned figures, and especially in reference to figure 1, we can appreciate a view of the group on the basis of a preferred use of the instrument of the invention, which involves a principal cylindrical plunger movable along the axis (1) within a cover (2), also cylindrical and concentric with the same. This cover (2) has a projection (4) which permits placement of the instrument end to end against the external genitals, to then manually slide the plunger (1), taking it by its strangled end portion (5) until its other end comes up against cervix of the uterus of the patient, or in the absence of this, against the vaginal vault. In this position, the intravaginal graduated ring is set to zero, which is always visible because of a dark tonality of the same.

When the instrument has been placed in position, the patient is ordered to push and the quantity of centimeters is observed which this effort moves, the measurement is repeated and the greatest measurement will be recorded.

Figures 2, 3 and 4 show in detail and separately the elements that make up the instrument of the invention. Figure 1A shows us a variation of the instrument of the invention in which a cylindrical plunger (6) similar to the previous one is shown, but it is empty and opened subsequently, in the interior of which a extended blade (8) is placed which extends from this plunger and contains the widest part graduated in centimeters. The mentioned plunger (6) is movable within a cover (7), which is also cylindrical and which presents a prominent anterior end with the purpose of coming end to end with the external genitals of the patient. The procedure for placement of the instrument is the same described previously with the sole difference that the graduated blade (9) is manually adjusted to zero with the distal external end of the cover (7). When the patient pushes, she moves the plunger (6) which moves together with the graduated scale, permitting very precise observation of the advance of this plunger (6). The figures 2A, 3A and 4A, show in detail the elements that make up this variation of the instrument of this invention.

In reference to figure 1B, the same shows us another variation of the instrument of the invention, always under the same operating principle and only removing the form of quantification of the movement of the plunger by the patient. In effect, this variation involves the disposable cylindrical plunger (10) for intravaginal insertion end to end or connected to the rest of the plunger shaft (13) which involves a dentated portion which when it slides in an axial form moves a needle (16) in radial direction on a semicircular dial or graduated scale (15). This needle (16) and graduated scale (15) are solidary with the cylindrical cover (11) within which the plunger (11) slides in an axial manner, presents a handle (12) which facilitates the manipulation of the instrument against the external genitals of the patient. In this case, the instrument is placed in the same way as has been described, the intravaginal portion (10) is inserted end to end with the cervix of the uterus of the patient and the shaft is made to advance until it hits this plunger (10). Then the dentated portion is connected to the pinion of the needle (16), which has been placed at zero. When the group of the plunger has been moved by the effort of the patient, the dentated part of the axis (13) will also be shifted, making the needle turn, which will quantify the movement of the plunger in the graduated scale.

Figures 2B, 3B and 4B show in detail the pieces that make up this variation of the instrument of the invention. It should be noted that in the first two versions of the apparatus disposable segments of the plunger may be incorporated, connecting with or end to end with the rest of the same. Thus, other variations not contemplated herein may be attempted, provided that they shall be circumscribed to the field and scope of the idea herein described.

This study surveys a total of 110 women, on whom the Utero-Vaginal drop measurement was applied. Twenty (20) patients (18.18%) have not given birth to children, ninety (90) patients (81.82%) have a midpoint of 2.2 parturitions.

Patients with lengthy labor, forceps application, perineal, torn, infected or weakened tissue in episotomies were excluded from this study.

Importance was given to an Hysterometric Uterine position, Vaginal examination, and Echosonography. Patients with a fibromatose uterus, measuring more than 8 cm and with evidence of genital prolapse were excluded.

**Age:**

The age midpoint was 32, the range extending from 18 to 45
Under 20, 25 patients = 22.72%
From 20 to 29, 25 patients = 31.81%
From 30 to 39, 28 patients = 25.45%
From 40 to 49, 22 patients = 20.00%
It can be observed that the first two groups constitute 54.5% and are under 30 years of age, and the first three groups, under 40 years of age, represent 80%.

**Parturitions:**

Twenty (20) patients (18.18%) had never given birth to a child, 20 patients (18.18%) had had Cesarean sections, and 70 patients (63.63%) had delivered from one to three times.

The midpoint for parturitions was 2.2, all carried out in hospitals.

The drop measurements obtained from the 110 patients checked in the range from 0.2 cm to 2 cm, were subdivided into two groups; one with the uterus in normal anteversoflexion position (AVF) and the other with uterus in retroversion (RVF).

The AVF group contained 80 patients (72.77%) with a drop between 0.2 cm and 1.8 cm for a midpoint of 1.11 cm distributed as follows:
30 patients (37.5%) from 0.2 cm to 0.9 cm
39 patients (48.75%) from 1 cm to 1.5 cm
11 patients (13.75%) from 1.6 cm to 1.8 cm
We observe that 69 patients (86.25%) have a drop between 0.2 cm and 1.5 cm, coinciding with the first two groups. While 11 patients (13.75%) correspond to a drop between 1.6 cm and 1.8 cm.

In the R.V.F. group, 20 patients (27.27%) had a drop between 1.2 cm and 2.0 cm for a midpoint of 1.67 cm, distributed as follows:
7 patients (23.33%) from 1.2 cm to 1.5 cm
23 patients (6.66%) from 1.6 cm to 2.0 cm
We observe that 76.66% had a drop between 1.6 cm and 2.0 cm.

A breakdown of the R.V.F. position shows the following:

| RVF | I | II | III |
|---|---|---|---|
| 7 patients (1.2-1.5cm) | 5(16.6%) | 2(6.66%) | |
| 23 patients (1.6-2.0cm) | | 18(60%) | 5(16.6%) |

Comparing the maximum drop of 1.8 cm from the A.V.F. group with the maximum drop of 2.0 cm from the RVF group, we obtain a difference of 0.2 cm. Taking into account the midpoint of 1.11 cm for the AVF group and the midpoint of 1.67 cm for the RVF group we obtain a difference of 0.56 cm. We thus draw the conclusion that the retroversion group has a more pronounced drop or mobility.

On the basis of what has been stated above, we obtain a maximum drop of 2.0 cm,and a midpoint of 1.21 cm for women without evidence of vaginal prolapse.

William Mengert (10) in 1935 has been the only person in the world to study this issue, measuring uterus drops and quantifying in centimeters lesions by section of the uterine ligamental system. His results were as follows:

1. Sectioning of the Round Ligament affects the position to retroversion. The midpoint drops caused by the sectioning of the Round Ligaments was 0.3 cm, within a range from 0.0 cm to 0.5 cm.

2. Sectioning of the Parametrium:

   A. Upper third, midpoint drop was 0.1 cm; with a minimum of 0.0 cm and a maximum of 0.25 cm.
   B. Lower two-thirds, midpoint drop was 3.6 cm with a minimum of 0.5 cm and a maximum of 4.5 cm.

3. Sectioning of Paravaginal Ligaments:

   A. Upper third midpoint drop was 2.6 cm with a minimum of 1.25 cm. and a maximum of 6.0 cm.
   B. Middle third midpoint drop was 4.3 cm. with a minimum of 2.5 cm and a maximum of 6.0 cm.

The addition for the midpoint drop of the Paravaginal Ligaments gives a total of 6.9 cm and this quantity added to the 3.6 cm from the two lower thirds of the Parametrium midpoint drops, yields a total of 10.5 cm, which is equivalent to 100%.

4. Section of the Utero Sacrum Ligaments - the midpoint drop is 1.1 cm with a minimum of 0.0 cm and a maximum of 4.5 cm.

5. Section of the Pubocervical Ligament yields a midpoint drop of 0.1 cm with a minimum of 0.0 cm and a maximum of 0.4 cm.

6. <u>The Pelvic floor,</u> even though <u>it has never been sectioned</u> did not hinder the experimental prolapse of the uterus, <u>and was not an impediment to the descent, thus placing in doubt the role that other authors have given it as support of the uterus</u>.

We point out the importance of the Paravaginal Ligament shown by this study, since its drop was 65.71%, compared to 34.28% from the two lower thirds of the Parametrium Ligament.

The addition of both Paravaginal and Parametrium ligamental sections gives a total prolapse of 10.5 cm, equivalent to 100%.

The Uterus Sacrum's part in the uterine drop gives a 10.47% midpoint for 1.1 cm, which means that it plays a secondary role.

When the results of Mengert's work have been disseminated and analyzed, they will be compared and interpolated with the method obtained from living patients. The total Prolapse obtained by Mengert was 10.5 cm. for a 100% drop and each centimeter corresponds to a 9.5% drop.

A uterus in retroversion with elongated Round Ligaments has a 0.3 cm (or 2.85%) midpoint drop according to Mengert, in a retroversion the coincidence of the uterine axis with the vaginal axis and the lack of support of the cervix on the lavators plate would produce, in the face of mounting intra-abdominal pressure, a uterus drop thus elongating the Uterus Sacrum Ligaments, which in turn will be separated during a (Type II, Type III) retroversion by the uterus' body and as a result will become flaccid. Facilitating the descent of the uterus and vagina through levators hiatus. Therefore, the Round and Uterus Sacrum Ligaments would give:

$$0.3 \text{ cm} + 1.1 \text{ cm} = 1.4 \text{ cm} = 13.3\% \text{ drop}$$

The strains to which the Parametrium and Paravaginals will be subject, during uterus drop, will elongate them. Knowing that the Parametrium's variation has a minimum of 0.5 cm (4.76%) and a maximum of 4.5 (42.85%) with a midpoint of 3.6 cm (34.28%), we can infer that the previous 1.4 cm from U/S + R = 0.5 from the Parametrials generate a drop of 1.9 cm (18.05%).

If we add the midpoint Uterus Sacrum and Rounds elongation to the midpoint of the Parametrium's elongation, we find:

$$1.4 \text{ cm} + 3.6 \text{ cm} = 5.0 \text{ cm} = 47.6\%$$

If the uterus continues in the drops, and we add the 1.25 cm minimum Pravaginal elongation, we find:

$$0.5 \text{ cm} + 1.25 \text{ cm} = 6.25 \text{ cm} = 59.52\%$$

If we add the midpoint of the elongation of the Paravaginal to the Uterus Sacrum, Rounds and Parametrium midpoints, we find:

$$4.3 \text{ cm} + 1.1 \text{ cm} + 0.4 \text{ cm} + 3.6 \text{ cm} + 3.6 \text{ cm} = 9.4 \text{ cm} = 89.52\%$$

If we have a complete elongation of the principal support systems with a 6.9 cm (65.71%) for Paravaginals and 3.6 cm (34.28%) for the Parametrium, we have 6.9 cm + 3.6 cm = 10.5 cm = 100% drop.

Symmonds and Pratt from the Mayo Clinic (11) established the classification of the Vaginal Vault Prolapse in three groups:

I. Pure Entereocele with a relatively well supported vagina;

II. (the most frequent) Enterocele with Cictocoele and Rectocoele associated with a moderate Vaginal Vault Prolapse occasioned by defects in the vaginal walls; III. Complete vaginal eversion.

Traditionally, the uterine prolapse is classified according to the drop. Type I, the drop does not go further than the Vaginal Introitus; Type II,the uterine cervix protrudes partially or totally beyond the introitus, Type III, the uterus' total Prolapse is accompanied by a total vaginal eversion also termed Procidentia.

Obviously, the classification of Type II or III is easy to diagnose clinically during a good gynaecological exam, but this is unlike the case in Type I, which can pass undetected. Based on all previous explanations and on the results from the study of drops in health women who have not had Vaginal Prolapse or Tumoral Pathology of the uterus or annexes, with an AVF/RVF uterus, the following classification is defined:

Type I.

Normal.

I.A. Uterine or Vault drop from 0.0 to 2.0 cm. Incidence 9.52% to 19.09%, Midpoint 1.0 cm, e.g. Fig. 7

Pathological

I.B. Uterine or Vault drop from 2.01 cm to 5.0 cm. Incidence 19.14% to 47.61%, Midpoint 3.51 cm, e.g. Fig. 8

I.C. Uterine or Vault drop from 5.1 cm to 5.9 cm. Incidence 46.61% to 56.19%, Midpoint 5.45 cm, e.g. Fig. 9

I.D. Uterine or Vault drop from 6.0 cm to 7.15 cm. Incidence 57.14% to 68.09%, Midpoint 6.57 cm, e.g. Fig. 10

Type II

Uterine or Vault drop from 7.2 cm to 8.5 cm. Incidence 68.57% to 80.95%, Midpoint 7.85%

Type III

Uterine or Vault drop from 8.5 cm to 10.5 cm. Incidence 81.90% to 100%, Midpoint 9.55 cm

Correlation: Classification and Ligamental Lesion:

1.A. Elastic and normal distension

$$0.1 - 2.0 \text{ cm } X = 1.0 \text{ cm}$$

I.B. Lesion: Round Ligaments + Utero Sacral Ligaments + Parametrium (midpoint)

2.01 - 5.0 cm X = 3.51 cm

I.C. Lesion: Round Ligaments + Utero Sacral Ligaments + Parametrium (minimum to maximum)

5.1 - 5.9 cm X = 5.45 cm

I.D. Lesion: Round Ligaments + Utero Sacral Ligaments + Parametrium (maximum) + Paravaginal (minimum)

6.0 - 7.15 cm X = 6.57 cm

II. Lesion: Round Ligaments + Utero Sacral + Parametrium (maximum) + Paravaginal (midpoint)

7.2 - 8.5 cm X = 7.85 cm

III. Lesion: Parametrium (midpoint) + Paravaginal (midpoint)

8.6 - 10.5 cm X = 9.5 cm

**Claims**

1. An intra-vaginal diagnostic instrument comprising a plunger (1, 6, 10) for insertion into the vagina of a patient, a sleeve (2, 7, 11) within which the plunger (1, 6, 10) is slidable and a scale (3, 9, 15) which co-operates with an indicating means (20, 16) indicating the movement of the plunger (1, 6, 10) in relation to the sleeve (2, 7, 11), the plunger (1, 6, 10) having a first end which is placeable against the cervix of the uterus or the vaginal vault of the patient in which position the indicating means (20, 16) is adjustable to indicate an initial reading on the scale (3, 9, 15), wherein the plunger (1, 6, 10) is moveable by a force exerted by the patient on the first end of the plunger (1, 6, 10) providing a subsequent reading of the indication means (20, 16) on the scale (3, 9, 15).

2. An intra-vaginal diagnostic instrument as claimed in Claim 1, wherein the sleeve (2, 7, 11) has a projection (4) for location against the external entrance to the vagina.

3. An intra-vaginal diagnostic instrument as claimed in Claim 1 or Claim 2, wherein the indicating means (20) is in the form of a notch provided on the plunger (1).

4. An intra-vaginal diagnostic instrument as claimed in Claim 1 or Claim 2, wherein the indicating means is in the form of an end of the sleeve (7) distal from the entrance to the vagina.

5. An intra-vaginal diagnostic instrument as claimed in Claim 1 or Claim 2, wherein a shaft (14) is provided having a dentated part which when moved axially due to movement of the plunger (10) moves a needle (16) in a radial direction on a scale (15), wherein the scale (15) and needle (16) are attached to the sleeve (11).

6. An intra-vaginal diagnostic instrument as claimed in Claim 5, wherein the plunger (10) is disposable and is placed end to end with the shaft (14).

7. An intra-vaginal diagnostic instrument as claimed in any one of the preceding Claims, wherein the scale (3, 9, 15) indicates a movement of the plunger (1, 6, 10) of at least 8 cm.

**Patentansprüche**

1. Intravaginales Diagnostikinstrument, bestehend aus einem Kolben (1, 6, 10) zur Einfügung in die Vagina einer Patientin, einer Hülse (2, 7, 11), innerhalb welcher der Kolben (1, 6, 10) verschiebbar ist, und einer Skala (3, 9, 15), die mit einer Anzeigeeinrichtung (20, 16) zusammenwirkt, welche die Bewegung des Kolbens (1, 6, 10) relativ zu der Hülse (2, 7, 11) anzeigt, wobei der Kolben (1, 6, 10) ein erstes Ende hat, das gegen den Hals der Gebärmutter oder die Vaginalwölbung der Patientin verschiebbar ist, in welcher Position die Anzeigeeinrichtung (20, 16) zur Anzeige einer anfänglichen Ablesung an der Skala (3, 9, 15) einstellbar ist, wobei der Kolben (1, 6, 10) durch eine durch die Patientin auf das erste Ende des Kolbens (1, 6, 10) ausgeübte Kraft bewegbar ist, um eine nachfolgende Ablesung der Anzeigeeinrichtung (20, 16) an der Skala (3, 9, 15) zu schaffen.

**2.** Intravaginales Diagnostikinstrument nach Anspruch 1, bei welchem die Hülse (2, 7, 11) einen Vorsprung (4) zur Lokalisierung gegen den äußeren Eintritt zu der Vagina hat.

**3.** Intravaginales Diagnostikinstrument nach Anspruch 1 oder Anspruch 2, bei welchem die Anzeigeeinrichtung (20) die Form einer an dem Kolben (1) vorgesehenen Kerbe hat.

**4.** Intravaginales Diagnostikinstrument nach Anspruch 1 oder Anspruch 2, bei welchem die Anzeigeeinrichtung die Form eines Endes der Hülse (7) fern von dem Eintritt zu der Vagina aufweist.

**5.** Intravaginales Diagnostikinstrument nach Anspruch 1 oder Anspruch 2, bei welchem ein Schaft (14) mit einem gezahnten Teil vorgesehen ist, der bei einer axialen Bewegung als Folge der Bewegung des Kolbens (10) eine Nadel (16) in einer radialen Richtung an einer Skala (15) bewegt, wobei die Skala (15) und die Nadel (16) an der Hülse (11) befestigt sind.

**6.** Intravaginales Diagnostikinstrument nach Anspruch 5, bei welchem der Kolben (10) wegwerfbar ist und endseitig an dem Schaft (14) angeordnet ist.

**7.** Intravaginales Diagnostikinstrument nach einem der vorhergehenden Ansprüche, bei welchem die Skala (3, 9, 15) eine Bewegung des Kolbens (1, 6, 10) von wenigstens 8 cm anzeigt.

**Revendications**

**1.** Instrument de diagnostic intra-vaginal comprenant un piston (1, 6, 10) pour insertion dans le vagin d'une patiente, une enveloppe (2, 7, 11) à l'intérieur de laquelle le piston (1, 6, 10) peut coulisser et une échelle (3, 9, 15) qui coopère avec un moyen indicateur (20, 16) indiquant le mouvement du piston (1, 6, 10) par rapport à l'enveloppe (2, 7, 11), le piston (1, 6, 10) ayant une première extrémité destinée à être placée contre le col de l'utérus ou la voûte vaginale de la patiente, position dans laquelle le moyen indicateur (20, 16) est ajustable pour indiquer une lecture initiale sur l'échelle (3, 9, 15), dans lequel le piston (1, 6, 10) est déplaçable par une force exercée par la patiente sur la première extrémité du piston (1, 6, 10) produisant une lecture ultérieure du moyen indicateur (20, 16) sur l'échelle (3, 9, 15).

**2.** Instrument de diagnostic intra-vaginal selon la revendication 1, dans lequel l'enveloppe (2, 7, 11) comprend une projection (4) destinée à être logée contre l'orifice externe du vagin.

**3.** Instrument de diagnostic intra-vaginal selon l'une des revendications 1 ou 2, dans lequel le moyen indicateur (20) a la forme d'une encoche ménagée sur le piston (1).

**4.** Instrument de diagnostic intra-vaginal selon l'une des revendications 1 ou 2, dans lequel le moyen indicateur a la forme d'une extrémité de l'enveloppe (7) distale par rapport à l'orifice d'entrée du vagin.

**5.** Instrument de diagnostic intra-vaginal selon l'une des revendications 1 ou 2, dans lequel une tige (14) est prévue avec une partie dentée qui, lorsqu'elle est déplacée axialement par le mouvement du piston (10), déplace une aiguille (16) dans une direction radiale sur une échelle (15), dans lequel l'échelle (15) et l'aiguille (16) sont solidaires de l'enveloppe (11).

**6.** Instrument de diagnostic intra-vaginal selon la revendication 5, dans lequel le piston (10) est amovible et est placé bout à bout avec la tige (14).

**7.** Instrument de diagnostic intra-vaginal selon l'une quelconque des revendications précédentes, dans lequel l'échelle (3, 9, 15) indique un mouvement du piston (1, 6, 10) sur au moins 8 cm.

Fig. 1

Fig. 2

Fig. 3

Fig. 4

EP 0 428 793 B1

Fig. 1-A

Fig. 2-A

Fig. 3-A

Fig. 4-A

EP 0 428 793 B1

Fig. 1-B

Fig. 2-B

Fig. 3-B

Fig. 4-B

EP 0 428 793 B1